(19) Europäisches Patentamt / European Patent Office / Office européen des brevets

(11) **EP 1 671 251 B1**

(12) **EUROPÄISCHE PATENTSCHRIFT**

(45) Veröffentlichungstag und Bekanntmachung des Hinweises auf die Patenterteilung:
**24.08.2016 Patentblatt 2016/34**

(21) Anmeldenummer: **04765479.3**

(22) Anmeldetag: **22.09.2004**

(51) Int Cl.:
*G06F 19/00* *(2011.01)*

(86) Internationale Anmeldenummer:
**PCT/EP2004/010607**

(87) Internationale Veröffentlichungsnummer:
**WO 2005/033982 (14.04.2005 Gazette 2005/15)**

(54) **VERFAHREN ZUR SIMULATION DER WECHSELWIRKUNG VON CHEMISCHEN SUBSTANZEN MIT LEBENDEN ORGANISMEN**

METHOD FOR SIMULATING THE INTERACTION OF CHEMICAL SUBSTANCES AND LIVING ORGANISMS

PROCEDE POUR SIMULER L'INTERACTION DE SUBSTANCES CHIMIQUES AVEC DES ORGANISMES VIVANTS

(84) Benannte Vertragsstaaten:
**AT BE BG CH CY CZ DE DK EE ES FI FR GB GR HU IE IT LI LU MC NL PL PT RO SE SI SK TR**

(30) Priorität: **02.10.2003 DE 10345836**

(43) Veröffentlichungstag der Anmeldung:
**21.06.2006 Patentblatt 2006/25**

(73) Patentinhaber: **Bayer Intellectual Property GmbH
40789 Monheim (DE)**

(72) Erfinder:
• **WILLMANN, Stefan
40593 Düsseldorf (DE)**
• **SCHMITT, Walter
41470 Neuss (DE)**
• **FOIS, Franco
40789 Monheim (DE)**

(74) Vertreter: **BIP Patents
c/o Bayer Intellectual Property GmbH
Alfred-Nobel-Straße 10
40789 Monheim am Rhein (DE)**

(56) Entgegenhaltungen:
• NESTOROV IVAN: "Whole body pharmacokinetic models." CLINICAL PHARMACOKINETICS. 2003, Bd. 42, Nr. 10, 18. August 2003 (2003-08-18), Seiten 883-908, XP008053606 ISSN: 0312-5963
• KLEIN MICHAEL T ET AL: "BioMOL: a computer-assisted biological modeling tool for complex chemical mixtures and biological processes at the molecular level." ENVIRONMENTAL HEALTH PERSPECTIVES. DEC 2002, Bd. 110 Suppl 6, Dezember 2002 (2002-12), Seiten 1025-1029, XP008053602 ISSN: 0091-6765
• GREENWOOD R ET AL: "THE KINETICS OF INSECTICIDE ACTION. PART IV: THE IN-VIVO DISTRIBUTION OF PYRETHROID INSECTICIDES DURING INSECT POISONING" PESTICIDE SCIENCE, ELSEVIER APPLIED SCIENCE PUBLISHER. BARKING, GB, Bd. 30, Nr. 1, 1990, Seiten 97-121, XP009028368 ISSN: 0031-613X
• STEUDLE ERNST: "Water transport across plant tissue: Role of water channels" BIOLOGY OF THE CELL (PARIS), Bd. 89, Nr. 5-6, August 1997 (1997-08), Seiten 259-273, XP002351491 ISSN: 0248-4900
• BIRTA L G ET AL: "SIMPAK-an interactive/graphics program for continuous-simulation system", SIMULATION USA, vol. 37, no. 4, October 1976 (1976-10), pages 115-122, XP008174355, ISSN: 0037-5497

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Anmeldung betrifft ein Computerverfahren zur Berechnung des pharmakokinetischen und pharmakodynamischen Verhaltens chemischer Substanzen in lebenden Organismen, z.B. in Säugetieren, Insekten oder Pflanzen. Die Raten und Ausmaße von Aufnahme, Verteilung, Metabolismus und Ausscheidung chemischer Substanzen in lebenden Organismen sind von großer Bedeutung z.B. im Bezug auf die *in vivo* Aktivität von pharmazeutischen Wirkstoffen und Pflanzenschutzmitteln oder für die toxikologische Risikoabschätzung. Computerverfahren wie das physiologisch-basierte pharmakokinetische (PB-PK) und pharmakodynamische (PD) Modelling sind geeignet, Wechselwirkungsprozesse von chemischen Substanzen mit lebenden Organismen zu beschreiben. Die Erfindung nach Anspruch 1 ermöglicht eine besonders einfache Generierung solcher Modelle auf der Basis einer Multi-Ebenen Architektur, in der einzeln eingekapselte Module, die z.B. den Organismus, die Substanz, die Applikationsform oder die Wirkform beschreiben, dynamisch miteinander verbunden werden. Durch Verbindung dieser Module zu einem Gesamtmodell wird ein System von gekoppelten Differentialgleichungen (Massenerhaltungsgleichungen) erzeugt, welches von einem Solver numerisch gelöst wird. Der besondere Vorteil der Erfindung liegt in der hohen Flexibilität des modularen Computerverfahrens, durch welches sich eine Vielzahl komplexer physiologischer und biochemischer Szenarien auf besonders einfache Weise generieren lässt. Die zur Erzeugung dieses Gleichungssystems erforderlichen chemischen, biologischen, physiologischen und anatomischen Input-Parameter können entweder in einer oder mehreren angebundenen Datenbanken enthalten sein oder über eine graphische Benutzeroberfläche eingegeben werden.

Zahlreiche PB-PK- und PD-Modelle für verschiedene Organismen wie Säugetiere [R. Kawai, M. Lemaire, J. L. Steimer, A. Bruelisauer, W. Niederberger, M. Rowland: Physiologically based pharmacokinetic study on a Cyclosporin derivate, SDZ IMM 125. J. Pharmacokin. Biopharm. 22, 327-365 (1994); P. Poulin, F.P. Theil: Prediction of pharmacokinetics prior to in vivo studies. 1. Mechanism-based prediction of volume of distribution. J. Pharm. Sci. 291, 129-156, (2002); P. Poulin, F.P. Theil: Prediction of Pharmacokinetics prior to in vivo studies. II. Generic physiologically based pharmacokinetic models of drug disposition, J. Pharm. Sci. 91, 1358-1370 (2002)], Insekten [R. Greenwood, M. G. Ford, E. A. Peace, D. W. Salt: The Kinetics of Insecticide Action. Part IV: The in vivo Distribution of Pyrethroid Insecticides during Insect Poisoning. Pestic. Sci. 30, 97-121 (1990)] oder Pflanzen [N. M. Satchivi, E. W. Stoller, L. M. Wax, D. P. Briskin: A nonlinear dynamic simulation model for xenobiotik transport and whole plant allocation following foliar application. Pestic. Biochem. Physiol. 68, 67-84 (2000); N. M. Satchivi, E. W. Stoller, L. M. Wax, D. P. Briskin: A nonlinear dynamic simulation model for xenobiotik transport and whole plant allocation following foliar application. Pestic. Biochem. Physiol. 68, 67-84 (2000)] sind in der Literatur beschrieben worden. Solche Modelle werden erfolgreich eingesetzt, um das Verhalten chemischer Substanzen auf der Basis von physiologischen und anatomischen Informationen sowie substanzspezifischen physiko-chemischen und biochemischen Parametern zu beschreiben. Die gängige Vorgehensweise dabei ist, den zu beschreibenden Organismus in mehrere physiologische Kompartimente zu unterteilen. Häufig entsprechen diesen Kompartimenten einzelne Organe wie Leber, Muskel, oder Lunge. Diese Kompartimente sind durch physiologische Parameter wie z.B. Blutflussraten sowie Wasser-, Fett- und Proteinanteil charakterisiert [R. Greenwood, M. G. Ford, E. A. Peace, D. W. Salt: The Kinetics of Insecticide Action. Part IV: The in vivo Distribution of Pyrethroid Insecticides during Insect Poisoning. Pestic. Sci. 30, 97-121 (1990)].

Nestorov fasst den Stand der Technik im Feld der Bereitstellung von PBPK-Modellen zusammen; es beschreibt die Nutzung von Modulen zur Beschreibung des Organismus und Beschreibung der Substanz sowie die üblichen Schritte der Modellentwicklung: i) Spezifikation der Ganzkörpermodell- und Gewebemodellstruktur, ii) Aufschreiben der Gleichungen, iii) Parameterspezifikation und / oder -abschätzung. Nestorov beschreibt verschiedene Lösungen für diese Übersetzung des PBPK-Modells in Software-Sprache, insbesondere graphische "klick-and-drag"-Software und lehrt, dass das Schreiben der Diffenrentialgleichungen und High-Level Coding zu einer "klick-and-drag"-Software zu bevorzugen sind (NESTOROV IVAN: "Whole body pharmacokinetic models.", CLINICAL PHARMACOKINETICS. 2003, Bd. 42, Nr. 10, 18. August 2003 (2003-08-18), Seiten 883-908).

Klein et al. beschreibt eine Vorgehensweise für eine detaillierte kinetische Modelierung eines biochemischen Prozesses, in der die toxikologisch relevante biochemische Reaktionen und Reaktionsnetzwerke in Mass Balance Gleichungen konvertiert werden. Das erzeugte Model kann in das klassische Kompartiment-PBPK-Model für eine auf Molekularebene detaillierte Modellierung integriert werden (KLEIN MICHAEL T ET AL: "BioMOL: a computer-assisted biological modeling tool for complex chemical mixtures and biological processes at the molecular level.", ENVIRONMENTAL HEALTH PERSPECTIVES. DEC 2002, Bd. 110 Suppl 6, Dezember 2002 (2002-12), Seiten 1025-1029).

Greenwood et al. beschreibt die Entwicklung eines pharmakokinetischen Kompartiment-Model einer Larve von Spodoptera Littoralis, als ein System von linearen Differentialgleichungen, die während der Simulation gelöst werden. Das Model wird mit Hilfe eines Computer Graphics Programms zusammengestellt und iterativ durch den Vergleich der Prädiktion mit den experimentellen Daten verbessert (GREENWOOD R ET AL: "THE KINETICS OF INSECTICIDE ACTION. PART IV: THE IN-VIVO DISTRIBUTION OF PYRETHROID INSECTICIDES DURING INSECT POISONING", PESTICIDE SCIENCE, ELSEVIER APPLIED SCIENCE PUBLISHER. BARKING, GB, Bd. 30, Nr. 1, 1990, Seiten 97-121).

BIRTA et al. beschreibt ein Computersystem mit interaktiver Graphik; der Nutzer kann insbesondere die Parameterwerte innerhalb des Models ändern. Während einer Simulation werden die festgelegten Diffentialgleichungen mit den neuen Parametern gelöst (BIRTA L G ET AL: "SIMPAK-an interactive/graphics program for continuous-simulation system", SIMULATION USA, Bd. 37, Nr. 4, Oktober 1976 (1976-10), Seiten 115-122, ISSN: 0037-5497)

Um ein physiologie-basiertes Modell zu erhalten werden diese Kompartimente entsprechend den anatomischen Vorgaben des Organismus rechnerisch durch Massenerhaltungsgleichungen miteinander verbunden, wie dies im oben zitierten Stand der Technik detailliert erläutert wird.

Nach dem Stand der Technik ist diese mathematische Kopplung starr, d. h. die Differentialgleichungen sind hart miteinander verdrahtet. Dadurch ist ein einmal erzeugtes Modell hinsichtlich der Zahl seiner Kompartimente und deren physiologischer Verschaltung fixiert. Der Erfindung liegt die Aufgabe zu Grunde, ein flexibles Verfahren zu entwickeln, welches eine variable Anzahl von Kompartimenten und Modulen (z.B. den Organismus, die Substanz oder die Applikationsform betreffend) nach individuellen Vorgaben dynamisch miteinander verschaltet, das entsprechende physiologisch-basierte Modell automatisch generiert und anschließend numerisch löst.

Gelöst wird diese technische Aufgabe durch ein neuartiges modulares Programmierverfahren nach Anspruch 1. Das Verfahren basiert auf einer Bibliothek aus voneinander unabhängigen Modulen, die jeweils für sich abgeschlossen sind und verschiedene Funktionalitäten aufweisen (z.B. die Definition von Substanzeigenschaften oder Applikationsform oder die Beschreibung der Physiologie des zu untersuchenden Organismus, etc.). Erst während der Laufzeit des Programms werden diese Module dynamisch zu einem vollständigen Modell zusammengestellt. Das hier beschriebene Verfahren erlaubt damit erstmalig eine flexible Behandlung unterschiedlicher komplexer Szenarien ohne die Notwendigkeit, Änderungen auf der Ebene der Differentialgleichungen vorzunehmen.

Modulare Programmier-Konzepte sind vom Prinzip her aus anderen Anwendungsgebieten bekannt (siehe z.B. US 5,930,154 oder WO 00/65523), sie wurden aber bislang nicht für pharmakokinetische oder pharmakodynamische Fragestellungen herangezogen.

Gegenstand der Erfindung, durch die die genannte Aufgabe gelöst wird, ist ein Computer implementiertes Verfahren zur Ermittlung der Wechselwirkung einer oder mehrerer chemischer wirksamen Substanzen mit Organismen mittels eines Computersystems dadurch gekennzeichnet, dass

umfassend mindestens ein Substanz-Modul, ein Applikations-Modul und ein Organismus-Modul, wobei

das Substanzmodul physiko-chemische und/oder biochemische Informationen über die Substanz(en), dessen/deren Verhalten simuliert werden soll, enthält,

das Organismus-Modul physiologische und anatomische Informationen, die den Organismus charakterisieren, mit dem die Substanz wechselwirken soll, beinhaltet,

das Applikations-Modul Informationen über den Ort und das zeitliche Profil der Verabreichung der Substanz(en) beinhaltet,

und das Verfahren folgende Schritte umfasst:

A) Auswahl mindestens einer Substanz aus dem Substanzmodul und Analyse ihrer physiko-chemischen und/oder biochemischen Eigenschaften, insbesondere aus der Reihe Lipophilie, Löslichkeit, Proteinbindung, Molekülgröße (ausgedrückt als Molekülgewicht oder -volumen), pKa-Wert im Falle von Säuren oder Basen, metabolische Degradierungsrate und kinetische Konstanten von aktiven Transportern,

B) Aus dem Organismus-Modul Auswahl anatomischer und physiologischer Kompartimente des jeweiligen Organismus ausgewählt wenigstens aus: a) im Falle von Säugetieren oder Insekten: Lunge periphere Organe, Blutwege, bevorzugt arterielle und/oder venöse Blutwege, oder Blutflüssigkeit; b) im Falle von Pflanzen Xylem- und Phloemstrom und Wurzel und /oder Blatt und/oder Stängel

C) Beschreibung der Kompartimente in einem Rechenprogramm im Hinblick auf die Transportprozesse der Substanz von und zu den Kompartimenten, Abbauprozesse und Wirkprozesse durch Massentransportgleichungen und kinetische Reaktionsgleichungen zur Beschreibung des jeweiligen Prozesses

D) Beschreibung der Transportprozesse und gegebenenfalls der Abbauprozesse der wirksamen Substanz im Organismus, insbesondere bei intravenöser, peroraler, inhalativer oder subkutaner Verabreichung, in dem Rechenprogramm durch Massentransportgleichungen und kinetische Reaktionsgleichungen,

E) Verknüpfung der nach Schritt C) und D) ausgewählten Gleichungssysteme und insbesondere numerische Berechnung des entstehenden Systems gekoppelter Differentialgleichungen,

F) Bestimmung des Konzentrations-/Zeitverlaufs der Wirksubstanz in ausgewählten Kompartimenten.

wobei

- für das Rechenprogramm eine hierarchische Modell-Architektur bestehend aus Modulen, die unterschiedliche Funktionalitäten aufweisen, verwendet wird und mindestens ein das Substanz-Modul, das Applikations-Modul und das Organismus-Modul umfassen,
- die Module dynamisch während der Laufzeit des Verfahrens und hierarchisch durch das Computersystem miteinander verbunden werden,
- anschließend im Schritt E) ein System von gekoppelten Differentialgleichungen durch einen DGL-Erzeuger automatisch erzeugt wird, welches durch numerische Integration durch einen numerischen Solver gelöst wird und
- als Ergebnis Konzentrations/Zeit-Kurven der betrachteten Substanz(en) in

Kompartimenten des betrachteten Organismus geliefert werden.

Bevorzugt steht der Organismus bzw. die Organismen Säuretiere, aus der Reihe Mensch, Affe, Hund, Schwein, Ratte oder Maus, oder Insekten, insbesondere Raupen, oder Pflanzen dar.

Der Wirkstoff wird gemäß einer bevorzugten Ausführung intravenös, oral, topikal, subkutan, intraperitoneal, inhalativ über die Nase oder Lunge, oder intracerebrovaskulär (im Fall von Säugetieren), bzw. intra-haemolymphatisch, topikal, oder oral durch Fraß oder Schlundsonde (im Fall von Insekten) appliziert, oder die Applikation erfolgt als Spritzbrühe mit Aufnahme über Blätter oder Wurzeln (im Fall von Pflanzen).

Bevorzugt ist ein Verfahren, das dadurch gekennzeichnet, dass die physiologischen Parameter, die die Organismen beschreiben, zeitabhängige Parameter sind.

In einer bevorzugten Ausführung des Verfahrens werden die Parameter teilweise einer Datenbank entnommen werden, die an das Rechner-System angebunden ist.

Die physiologischen und anatomischen Parameter können in einer bevorzugten Ausführung mittels Zufallszahlen im Rahmen einer vorgegebenen statistischen Streuung variiert werden (Populationskinetik).

Besonders bevorzugt ist ein Verfahren, bei denen für das Rechenprogramm eine hierarchische Modell-Architektur bestehend aus Modulen, die unterschiedliche Funktionalitäten aufweisen, und mindestens zwei Module, die entweder chemische oder biologische Eigenschaften der Substanz beinhalten, oder physiologische oder anatomische Informationen über den Organismus beinhalten, oder Informationen über die Darreichungsform oder die Wirkart beinhalten, verwendet werden.

Eine besonders bevorzugte Variante des Verfahrens ist dadurch gekennzeichnet, dass mehrere Modelle von Organismen zu einem Gesamtmodell, insbesondere zur Simulation von Drug-Drug-Wechselwirkungen, Mutter-Foetus-Modell, kombiniertes Insekt-Pflanzen-Modell, zusammengefasst werden.

Kern des Verfahrens ist die Realisierung eines modularen Simulationskonzepts mit dynamischer Generierung eines Differentialgleichungs- (DGL) Systems und dessen Anwendung auf pharmakokinetische und pharmakodynamische Fragestellungen. Das Simulationskernel besteht dazu aus einer Bibliothek einzelner Module, die die folgenden Funktionalitäten beinhalten:

- Definition der physiko-chemischen Substanzeigenschaften (z.B. Lipophilie, Affinität zu Plasmaproteinen, Molekülgewicht)
- Anatomische und physiologische Beschreibung des Organismus (Säugetier, Insekt, Pflanze)
- Beschreibung der einzelnen Kompartimente (Organe), die den Organismus bilden
- Beschreibung der Applikationsform der chemischen Substanz (z.B. intravenös, oral, subkutan, topikal oder inhalativ für Säugetiere, oral oder topikal für Insekten, Applikation als Spritzbrühe und Aufnahme über Blätter oder Wurzel für Pflanzen)
- Zeitschleifen-Definition zur numerischen Integration
- Numerische Integration des Differentialgleichungssystems (z.B. nach dem Euler-Verfahren oder Runge-Kutta Verfahren)
- Speichern der Simulationsergebnisse (z.B. in einer Datei, Datenbank, o. ä.)

Diese individuellen Module werden dynamisch während der Laufzeit des Programms durch ein Computersystem, z.B. einen handelsüblichen PC, auf eine vordefinierte Weise hierarchisch miteinander verbunden. Anschließend wird automatisch ein System von gekoppelten DGLs (Massenerhaltungsgleichungen) erzeugt, welches durch numerische Integration gelöst wird. Als Ergebnis liefert das Modell Konzentrations/Zeit-Kurven der betrachteten chemischen Substanz in den diversen Kompartimenten des betrachteten Organismus, welche dann weiter z.B. zur Berechnung einer pharmakologischen Wirkung herangezogen werden können.

Das minimale Modell, welches dem Verfahren zu Grunde liegt, besteht mindestens aus einem Substanz-Modul, einem Applikations-Modul, einem Organismus-Modul, dem DGL-Generator und dem numerischen Solver für das DGL-System, sowie optionalen weiteren Modulen, z.B. einem pharmakodynamischen Wirkmodul.

Das Substanz-Modul beinhaltet physiko-chemische und/oder biochemische Informationen über die Substanz, deren Verhalten simuliert werden soll. Dies können z.B. Werte für die Lipophilie, Löslichkeit, z.B. Wasser oder intestinalem Fluid, Proteinbindung, z.B. von Plasmaproteinen, Molekülgröße (ausgedrückt als Molekülgewicht oder -volumen), pKa-Wert im Falle von Säuren oder Basen, metabolische Degradierungsraten, kinetische Konstanten von aktiven Transportern, etc. sein. Diese Parameter sind generell durch *in vitro* Experimente bestimmbar oder - in einzelnen Fällen, z.B. im Fall der Lipophilie - durch bekannte Vorhersagemodelle, die z.B. QSAR, HQSAR oder neuronalen Netze verwenden, direkt aus der Struktur berechenbar.

Das Organismus-Modul beinhaltet physiologische und anatomische Informationen, die den Organismus charakterisieren, mit dem die Substanz wechselwirken soll. Organismus-spezifische Informationen sind unter anderem Volumina und Volumenblutflussraten sowie Wasser-, Fett- und Proteingehalt der einzelnen Kompartimente (im Fall von Säugetieren und Insekten), oder Xylem- und Phloemtransportraten und die Volumina von Symplast, Apoplast und Vakuolen im Fall von Pflanzen. Diese physiologischen Parameter können zeitlich konstant sein oder auch zeitabhängig sein, um z.B. Wachstumsprozesse und andere physiologische Veränderungen mit der Zeit mit berücksichtigen zu können. Diese physiologischen und anatomischen Parameter sind für eine Vielzahl relevanter Organismen in der Literatur veröffentlicht.

Das Applikations-Modul beinhaltet Informationen über den Ort und das zeitliche Profil der Verabreichung der Substanz. Übliche Formen für die Verabreichung von pharmazeutischen Wirkstoffen bei Säugetieren sind die intravenöse Applikation als Bolus oder Infusion, orale Applikation als Lösung, Kapsel oder Tablette, subkutane Administration, intraperitoneale Administration, topikale Administration mit Aufnahme über die Haut oder Schleimhäute, sowie nasale oder inhalative Administration. Insekten nehmen chemische Substanzen vorwiegend oral oder über die Kutikula nach topikalem Kontakt auf. Die Substanzaufnahme in Pflanzen verläuft entweder über die Wurzeln oder über die Blätter.

Der DGL-Generator erzeugt automatisch des Differentialgleichungssystem basierend auf der Massenbilanz innerhalb des Organismus. Der Solver übernimmt die numerische Integration des DGL-Systems und liefert als Resultat Konzentrations-Zeit-Profile der chemischen Substanz in den Kompartimenten des Organismus. Diese Daten können weiter genutzt werden um z.B. eine pharmakologische Wirkung an einem Zielenzym (Target) zu beschreiben.

Zur dynamischen Verknüpfung der Module ist ein hierarchisches Management der Variablen, die in den Modulen enthalten sind, erforderlich, da verschiedene Variablen aus unterschiedlichen Modulen voneinander abhängen können. So werden z.B. die substanz-spezifischen physiko-chemischen Parameter (aus dem Substanz-Modul) zusammen mit physiologischen Informationen (aus dem Organismus-Modul) verwendet, um Gleichgewichts-Verteilungskoeffizienten zwischen Plasma und den peripheren Kompartimenten im Säugetierkörper zu berechnen. In ähnlicher Weise existieren Abhängigkeiten zwischen den einzelnen Kompartimenten innerhalb eines Organismus. Der Blutfluss in der Lunge ist beispielsweise gegeben durch die Summe der Blutflussraten aller übrigen Organe, da die Blutzirkulation im Säugetierkörper in sich geschlossen ist. Solche Abhängigkeiten erfordern eine generelle hierarchische Datenstruktur, damit sie automatisch erkannt und berücksichtigt werden können. Eine solche hierarchische Datenstruktur ist Teil der vorliegenden Erfindung. Die Module werden hierzu von einer Datenbank von Objekten verwaltet. Die Objektdatenbank kann dynamisch beliebig erweitert werden, was ein Höchstmaß an Funktionalität garantiert. Auch mehrere Module mit gleicher oder ähnlicher Funktionalität sind zulässig. Die Datenstrukturen für die mathematische Beschreibung des Gesamtmodells sind in den einzelnen Modulen eingekapselt. Eine Besonderheit dieses Konzeptes ist die modulübergreifende hierarchische Verknüpfung der Daten, die wiederum, ähnlich den Objektmodulen, von einer eigenen Datenbank verwaltet werden. Dadurch ist ein, modellbedingter, Zugriff auf Daten anderer Module möglich. Die Module des Simulationskernels müssen von einem Hauptprogramm generiert und in die gewünschte Reihenfolge verknüpft werden.

Zur Verwaltung der diversen Input-Parameter wurde zum einen eine Datenbankanbindung der Compound- und Organismus-Parameter realisiert. Darüber hinaus (oder auch als einzige Alternative) stellt ein graphisches User-Interface (GUI) sicher, dass alle relevanten Modell-Parameter (a) für den User sichtbar und (b) durch ihn editierbar sind. Das Verändern einzelner Daten in einem Modul führt zur Prüfung der Abhängigkeiten in den restlichen, entsprechend der Datenhierarchie, über vordefinierte Meldungen. Diese Prüfung der Daten-Abhängigkeiten ist wesentlicher Bestandteil des Modell-Generators, da sie zu jedem Zeitpunkt die Korrektheit des Modells sicherstellt. Über das GUI werden auch die Ergebniskurven sowie optional aus diesen Kurven abgeleitete typische pharmakokinetische Parameter (z.B. Fläche unter der Kurve, maximale Konzentration, Zeitpunkt der maximalen Konzentration, Halbwertszeit, etc.) oder pharmakodynamische Parameter (z.B. Wirkstärke und - dauer) angezeigt.

Der besondere Vorteil des Verfahrens nach Anspruch 1 liegt in der Flexibilität, mit der auf einfache Weise unterschiedliche und komplexe Modelle erzeugt werden können. Die folgenden Beispiele sollen dies anhand von Figuren illustrieren. Sie zeigen relevante Szenarien unterschiedlicher Komplexität. Die Beispiele sollen nicht als Limitierung der Anwendbarkeit der vorliegenden Erfindung verstanden werden.

In den Figuren zeigen:

Fig. 1      Die schematische Darstellung eines vier-Kompartiment Säugetiermodells

Fig. 2      Die schematische Darstellung eines physiologischen GanzkörperSäugetiermodells

Fig. 3      Die schematische Darstellung eines Organs im Ganzkörpermodell

Fig. 4      Die schematische Darstellung eines physiologischen Modells für Raupen

Fig. 5      Die schematische Darstellung eines physiologischen Pflanzenmodells

Fig. 6      Die schematische Darstellung des einfachsten Modell-Szenarios: Das Modell besteht aus den Modulen "Substanz", "Verabreichung", "Organismus", "DGL-Generator", und "Integrator". Optional kann ein "Aktions-Modul" betrachtet werden.

Fig. 7      Die schematische Darstellung der Anbindung des Simulationsmodells an Datenbanken und die graphische Benutzeroberfläche.

Fig. 8      Die schematische Darstellung eines Modells für multiple Administration

Fig. 9      Die schematische Darstellung eines Modells für eine Substanz, die einem enterohepatischen Kreislauf unterliegt

Fig. 10     Die schematische Darstellung eines Modells zur Beschreibung der Wechselwirkung zweier Substanzen

Fig 11a.    Die schematische Darstellung eines Modells zur Beschreibung der Wechselwirkung zweier Substanzen im Verhältnis aktiver Metabolit/Prodrug.

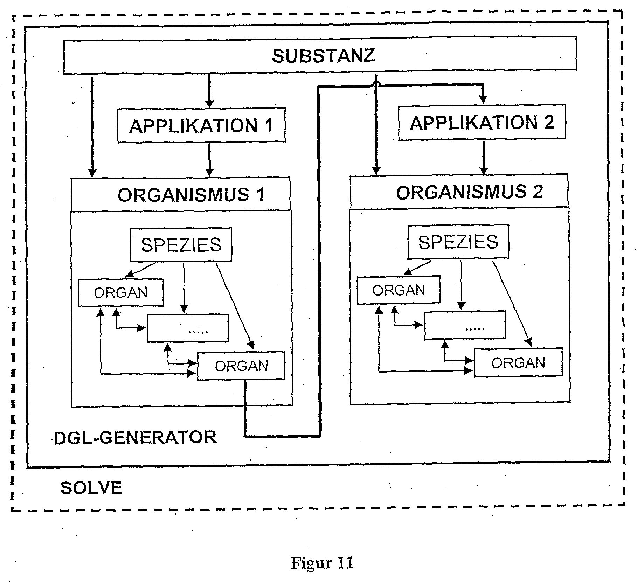

Fig. 11     Die schematische Darstellung eines Modells zweier miteinander in Wechselwirkung stehender Organismen. Beispiele für ein solches Szenario sind des Mutter-Foetus-Modell sowie das kombinierte Insekten-Pflanze-Modell.

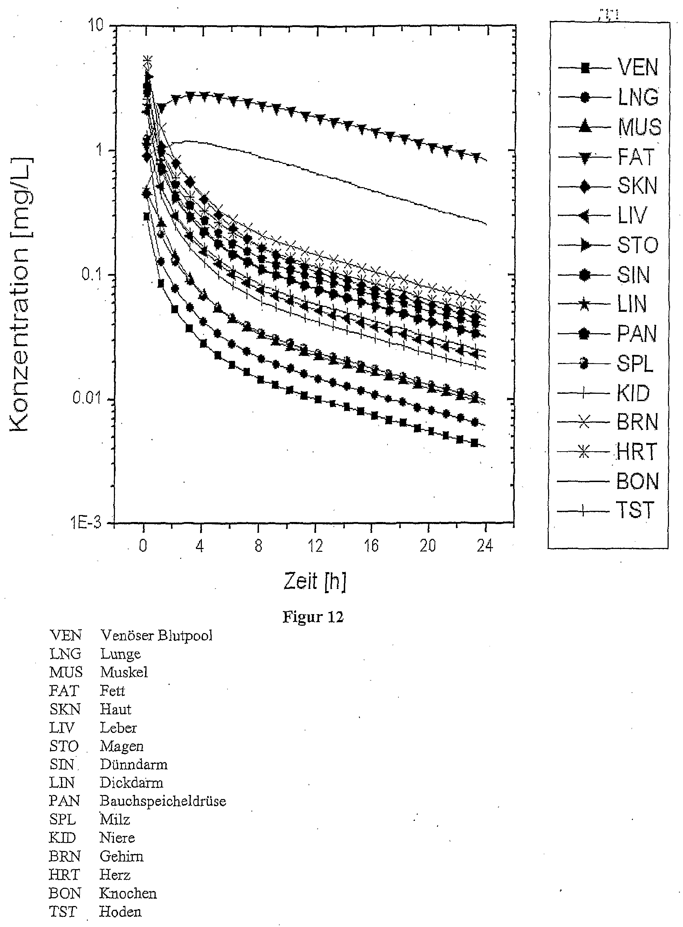

Fig. 12     den Konzentrations/Zeitverlauf einer Substanz in verschiedenen Organen einer Ratte

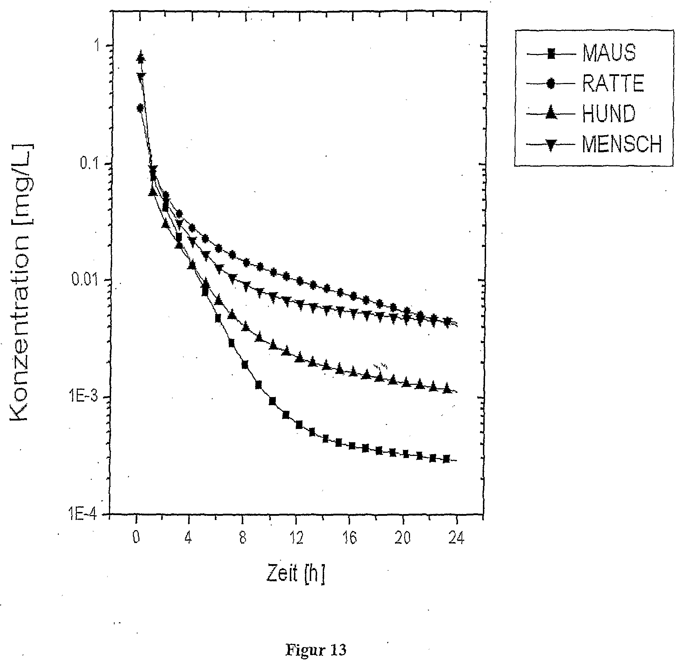

Fig. 13     den Konzentrations/Zeitverlauf einer Substanz im Plasma verschiedener Säuger

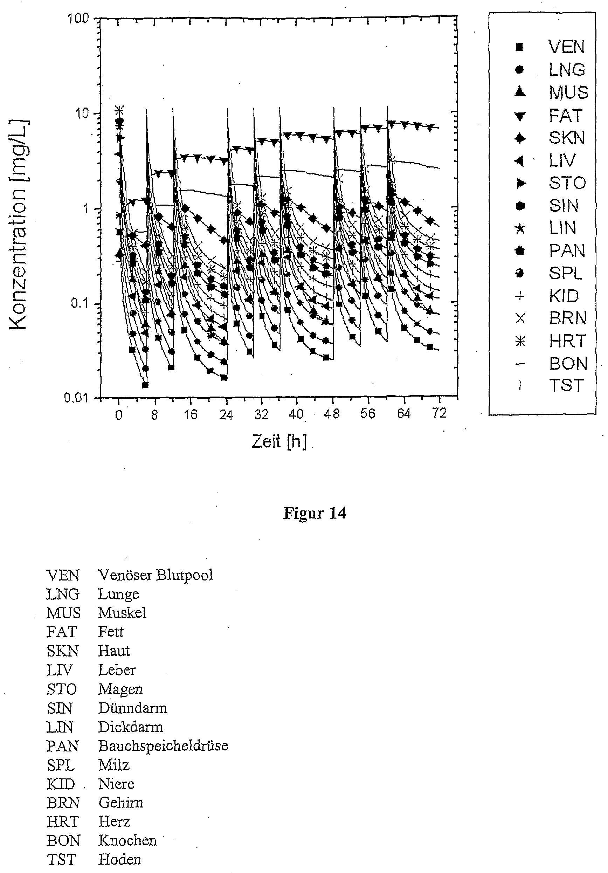

Fig. 14     den Konzentrations/Zeitverlauf einer Substanz in verschiedenen Organen eines Menschen

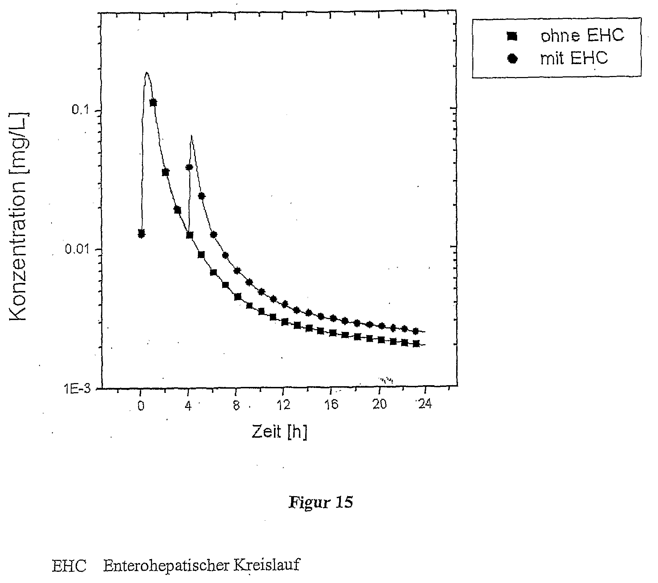

Fig. 15     den Konzentrations/Zeitverlauf einer Substanz nach peroraler Administration im Plasma eines Menschen

## Beispiele

In diesem Abschnitt werden bevorzugte Ausführungsformen für die zu beschreibenden Organismen (Säugetier, Insekt und Pflanze) beschrieben, anschließend wird deren Einbau in das dynamische Simulationsmodell an Beispielen gezeigt.

### *Beispiele für verschiedene Organismus-Module*

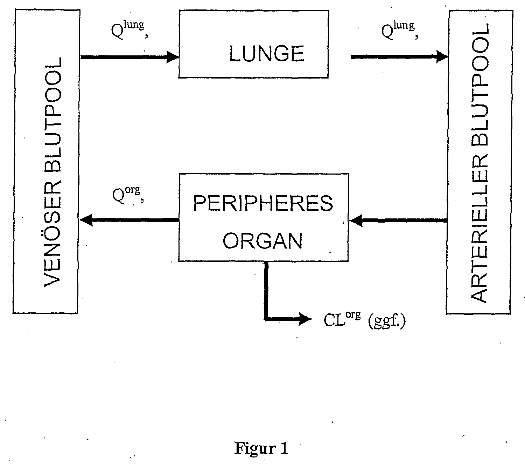

Figur 1 zeigt ein stark vereinfachtes vier-Kompartiment-Modell für einen Säugetierkörper. Der Säugetierkörper besteht hier lediglich aus venösen und arteriellen Blutpool, der Lunge und einem weiteren peripheren Kompartiment, über das die Substanz metabolisch eliminiert wird. Das periphere Kompartiment wird über den arteriellen Blutstrom (Blutflussrate $Q_{org}$) mit der Substanz (Blutkonzentration $c^{in}_{org}$) versorgt. Nach Wechselwirkung in dem Kompartiment strömt das venöse Blut (Konzentration der Substanz $C^{out}_{org}$) in die Lunge, über die der Blutkreislauf geschlossen ist ($Q_{lunge} = Qorg$).

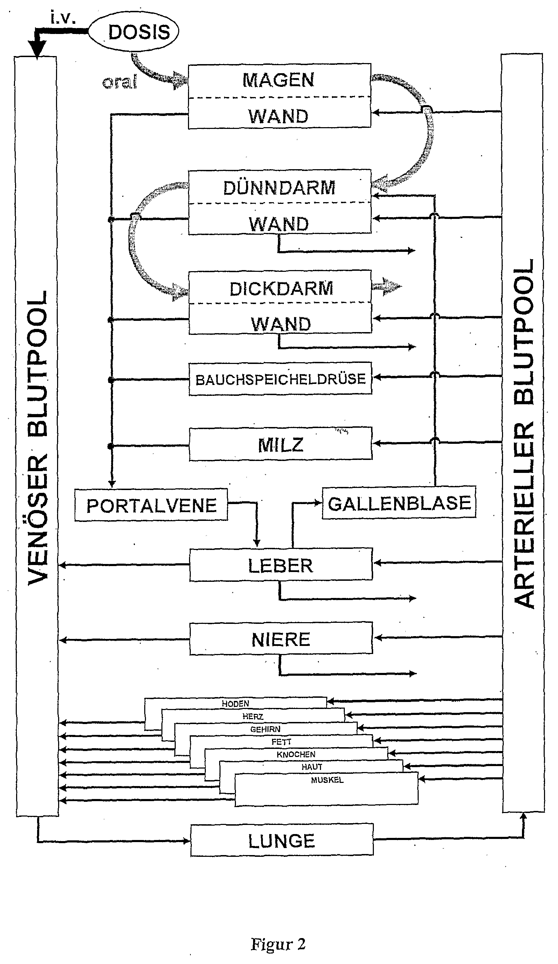

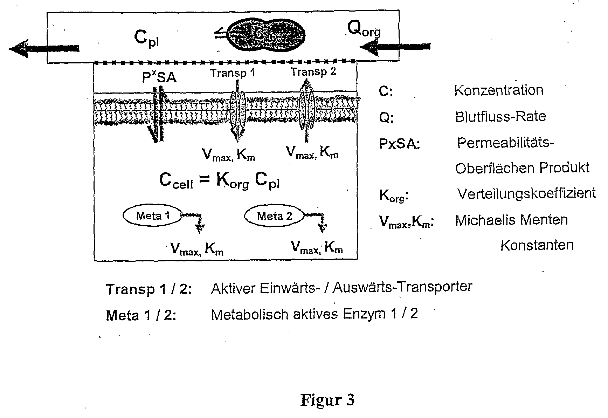

In einer bevorzugten Ausführungsform des Verfahrens wird als Organismus-Modul das in Figur 2 gezeigte Säugetiermodell verwendet. Der Säugetierkörper besteht hier ebenfalls aus dem arteriellen und dem venösen Blutpool, einem Lungen-Kompartiment und den folgenden peripheren Organen: Leber, Niere, Muskel, Knochen, Haut, Fett, Gehirn, Magen, Dünndarm, Dickdarm, Bauchspeicheldrüse, Milz, Gallenblase und Hoden. Diese Ausführungsform wird im folgenden als "Ganzkörper-Modell" bezeichnet. Jedes Organ in diesem Ganzkörper-Modell ist wiederum unterteilt in mehrere Subkompartimente, die jeweils den vaskulären Raum (bestehend aus Plasma und den roten Blutkörperchen), interstitiellen und intrazellulären Raum repräsentieren (Fig. 3). Plasma und interstitieller Raum werden als equilibriert behandelt. Der Substanztransport zwischen dem interstitiellen Raum und dem intrazellulären Raum wird beschrieben als passiver Diffusionsprozess, der einer Kinetik erster Ordnung folgt, oder als aktiver Transportprozess, der mittels einer sättigbaren Michaelis-Menten Kinetik modelliert wird. Analog existieren in jedem intrazellulären Subkomprtiment ein oder mehrere Michaelis-Menten-Terme, die dem metabolischen Abbau der Substanz Rechnung tragen.

Das resultierende System von gekoppelten Differentialgleichungen hat die folgende Form: Für jedes periphere Organ (oberer Index "org") existieren drei Differentialgleichungen für die Subkompartimente "Plasma" (pl), "Rote Blutkörperchen" bzw. "Blutzellen" (bc) und "Zellinneres" (cell). Das Ganzkörpermodell beinhaltet die folgenden peripheren Organe: Lunge, Magen, Dünndarm, Dickdarm, Pankreas, Milz, Leber, Niere, Gehirn, Herz, Muskel, Knochen, Haut, Fett und Hoden. Für das Plasma und den interstitiellen Raum (int) ergibt sich pro Organ:

**MASSENBILANZGLEICHUNG**

$$[f_{vas}^{org}(1-HCT)+f_{int}^{org}]\,V^{org}\,\frac{dC_{pl}^{org}}{dt}$$

$$= Q_{pl}^{org}\,(C_{pl}^{art}-C_{pl}^{org})$$

$$-\frac{PA_{bc}^{org}}{K_{pl}}\left(C_{pl}^{org}-\frac{C_{bc}^{org}}{K_{bc}}\right)$$

$$-\frac{PA^{org}}{K_{pl}}\left(C_{pl}^{org}-\frac{C_{cell}^{org}}{K^{org}}\right)$$

$$-\frac{V_{max,in}^{org}\,C_{pl}^{org}\,/\,K_{pl}}{K_{m,in}^{org}+C_{pl}^{org}\,/\,K_{pl}}$$

$$+\frac{V_{max,ex}^{org}\,C_{cell}^{org}\,/\,(K^{org}\,K_{pl})}{K_{m,ex}^{org}+C_{cell}^{org}\,/\,(K^{org}\,K_{pl})}$$

**BEDEUTUNG**

Inter-kompartimenteller Flussterm

Diffusiver Massentransport zu den roten Blutkörperchen

Diffusiver Massentransport in das Zellinnere

Aktiver Transportterm in das Zellinnere hinein (Influx)

Aktiver Transportterm aus dem Zellinneren heraus (Efflux)

Für die roten Blutkörperchen folgt:

**MASSENBILANZGLEICHUNG**

$$f_{vas}^{org}\,HCT\,V^{org}\,\frac{dC_{bc}^{org}}{dt}$$

$$= Q_{bc}^{org}\,(C_{bc}^{art}-C_{bc}^{org})$$

$$+\frac{PA_{bc}^{org}}{K_{pl}}\left(C_{pl}^{org}-\frac{C_{bc}^{org}}{K_{bc}}\right)$$

**BEDEUTUNG**

Inter-kompartimenteller Flussterm

Diffusiver Massentransport zu den roten Blutkörperchen

Das Zellinnere wird beschrieben mittels:

**MASSENBILANZGLEICHUNG**

$$f_{cell}^{org} \ V^{org} \ \frac{dC_{cell}^{org}}{dt} \ = \ \frac{PA^{org}}{K_{pl}} \left( C_{pl}^{org} - \frac{C_{cell}^{org}}{K^{org}} \right)$$

$$+ \ \frac{V_{max,in}^{org} \ C_{pl}^{org} \ / \ K_{pl}}{K_{m,in}^{org} + C_{pl}^{org} \ / \ K_{pl}}$$

$$- \ \frac{V_{max,ex}^{org} \ C_{cell}^{org} \ / \ (K^{org} \ K_{pl})}{K_{m,ex}^{org} + C_{cell}^{org} \ / \ (K^{org} \ K_{pl})}$$

$$- \ \frac{V_{max,M1}^{org} \ C_{cell}^{org} \ / \ (K^{org} \ K_{pl})}{K_{m,M1}^{org} + C_{cell}^{org} \ / \ (K^{org} \ K_{pl})}$$

$$- \ \frac{V_{max,M2}^{org} \ C_{cell}^{org} \ / \ (K^{org} \ K_{pl})}{K_{m,M2}^{org} + C_{cell}^{org} \ / \ (K^{org} \ K_{pl})}$$

$$- \ CL^{org} \ C_{cell}^{org}$$

**BEDEUTUNG**

Diffusiver Massentransport in das Zellinnere

Aktiver Transportterm in das Zellinnere hinein (Influx))

Aktiver Transportterm aus dem Zellinneren heraus (Efflux)

Metabolischer Abbau (Enzym 1)

Metabolischer Abbau (Enzym 2)

Abbau erster Ordnung (z.B. in der Leber oder Niere)

Die Lunge (org = lng) wird analog beschrieben, allerdings ist hier der Zustrom nicht über den arteriellen Blutpool (art) sondern über den venösen (ven):

**MASSENBILANZGLEICHUNG**

$$[f_{vas}^{lng}(1-HCT) + f_{int}^{lng}] \ V^{lng} \ \frac{dC_{pl}^{lng}}{dt} \ = Q_{pl}^{lng} \ (C_{pl}^{ven} - C_{pl}^{lng})$$

$$\pm \ ...$$

$$f_{vas}^{lng} \ HCT \ V^{lng} \ \frac{dC_{bc}^{lng}}{dt} \ = Q_{bc}^{lng} \ (C_{bc}^{ven} - C_{bc}^{lng})$$

$$\pm \ ...$$

**BEDEUTUNG**

Blutfluss ist umgekehrt im Vergleich zu den übrigen Organen !

analog den anderen Organen

Blutfluss ist umgekehrt im Vergleich zu den übrigen Organen !

analog den anderen Organen

Die aus den Organen herausströmenden Konzentrationen vereinigen sich im venösen Blutpool. Die resultierende Plasmakonzentration folgt aus dem blutfluss-gewichteten Mittelwert der einzelnen Organkonzentrationen:

**MASSENBILANZGLEICHUNG**       **BEDEUTUNG**

$$X \ V^{ven} \ \frac{dC_{pl/bc}^{ven}}{dt} \ = Q_{pl/bc}^{lng} \left( \sum_{org} Q_{pl/bc}^{org} \ C_{pl/bc}^{org} \ / \ \sum_{org} Q_{pl/bc}^{org} - C_{pl/bc}^{ven} \right)$$

| MASSENBILANZGLEICHUNG | BEDEUTUNG |
|---|---|
| $$\mp \frac{PA_{bc}^{ven}}{K_{pl}}\left(C_{pl}^{ven} - \frac{C_{bc}^{ven}}{K_{bc}}\right)$$ | Diffusiver Massentransport zwischen Plasma (-) und den roten Blutkörperchen (+) |
| | Plasma Clearance (ggf.) |
| | Input-Funktion für Applikation in den venösen Blutpool |

$$- CL_{ven}\, C_{pl}^{ven}$$

$$+ \frac{dIV_{pl}}{dt}$$

$$X = \begin{cases} 1 - HCT & \text{für Plasma} \\ HCT & \text{für rote Blutkörper chen} \end{cases}$$

Der arterielle Blutpool wird schließlich beschrieben durch:

| MASSENBILANZGLEICHUNG | BEDEUTUNG |
|---|---|
| $$X\, V^{art}\, \frac{dC_{pl/bc}^{art}}{dt} = Q_{pl/bc}^{\ln g}\,(C_{pl/bc}^{\ln g} - C_{pl/bc}^{art})$$ | |
| $$\mp \frac{PA_{bc}^{art}}{K_{pl}}\left(C_{pl}^{art} - \frac{C_{bc}^{art}}{K_{bc}}\right)$$ | Diffusiver Massentransport zwischen Plasma (-) und den roten Blutkörperchen(+) |

$$X = \begin{cases} 1 - HCT & \text{für Plasma} \\ HCT & \text{für rote Blutkörper} \end{cases}$$

In diesem Gleichungssystem bedeuten:

$f_x^{org}$ : Volumenanteil des vaskulären (x = vas), interstitiellen (x = int) oder intrazellulären Raums (x = cell) des Organs

$V^{org}$ : Volumen des Organs

HCT : Haematokrit (= Volumenanteil der roten Blutkörperchen im Vollblut)

$Q_x^{org}$ : Blutflussrate des Plasmas (x = pl) bzw. der Blutzellen (x = bc) im Organ

$PA_{bc}^{org}$: Permeabilitäts-Oberflächenprodukt der roten Blutkörperchen

$PA^{org}$ : Permeabilitäts-Oberflächenprodukt des Organs

$K_{pl}$ : Gleichgewichts-Verteilungskoeffizient der Substanz zwischen Plasma und Wasser

$K_{bc}$ : Gleichgewichts-Verteilungskoeffizient der Substanz zwischen roten Blutkörperchen und Plasma

$K^{org}$ : Gleichgewichts-Verteilungskoeffizient der Substanz zwischen dem Organ und Plasma

$V_{max}$, $K_m$: Michaelis-Menten-Konstanten für aktiven Transport bzw. Metabolismus

Alternativ zu oder in Kombination mit der hier beschriebenen intravenösen Applikation kann auch eine Aufnahme über die Magen-Darm-Schleimhaut nach peroraler Verabreichung simuliert werden. Die Lösungen dieses Gleichungssystems ergeben die Konzentrations-Zeit-Beziehungen für alle im Modell enthaltenen Kompartimente.

Zur Beschreibung einer pharmakologischen Wirkung kann weiterhin die Konzentrations-Zeit-Beziehung in dem Kompartiment, welches das biologische Target des Wirkstoffs enthält, mit einem pharmakodynamischen Effekt verknüpft werden. Typische Effektfunktionen sind z.B.:

- Hyperbolische oder sigmoide Emax-Modelle: $\text{Effekt} = E_0 + \dfrac{E_{max}\, C_x^{\gamma}}{EC_{50}^{\gamma} + C_x^{\gamma}}$

Effekt = pharmakologischer Wirkparameter (zeitabhängig)
Eo = Basiswert des pharmakologischen Wirkparameters
$E_{max}$ = Maximalwert der pharmakologischen Wirkung
$EC_{50}$ = Konzentration, bei der 50 % des maximalen Effekts erreicht sind
$C_x$ = Konzentration am Wirkort (zeitabhängig)
$\gamma$ = Formparameter

- Potenzfunktionen: $\text{Effekt} = E_0 + \beta\, C_x^{\gamma}$ bzw. Log-Linear Modelle:

$$\text{Effekt} = E_0 + \beta\, \text{Ln}(C_x)$$

Effekt = pharmakologischer Wirkparameter (zeitabhängig)
Eo = Basiswert des pharmakologischen Wirkparameters
$\beta$ = Parameter für die Steigung des Effekts als Funktion der    Konzentration
$C_x$ = Konzentration am Wirkort (zeitabhängig)
$\gamma$ = Formparameter

- Wirkstoffinteraktionsmodelle wie z.B. partieller oder vollständiger Antagonismus, etc.
- Kombinationen der vorgenannten Modelle, mit denen z.B. multiple Wirkzentren, oder Rezeptor-Transducer-Wechselwirkungen beschrieben werden können.

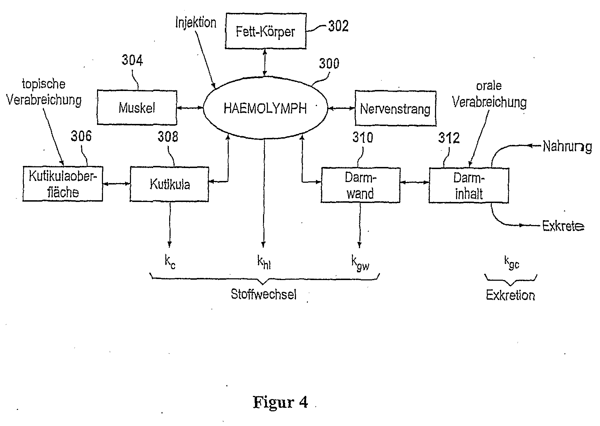

In einer weiteren bevorzugten Ausführungsform des Verfahrens repräsentiert das Organismus-Modul den Körperbau eines Insekts, insbesondere den einer Raupe (Figur 4). Der Raupenkörper besteht aus den folgenden Kompartimenten: Hämolymphe als zentrales Kompartiment, Kutikula, Muskel, Fettkörper, Nervensystem, und Darmwand als periphere Kompartimente, sowie den Kompartimenten Kutikula-Oberfläche und Darminhalt, über die ein Substanzaustausch mit der Umgebung stattfinden kann. Der interkompartimentelle Massentransport kann wiederum passiv via Diffusion oder mit Hilfe von Transportern als aktiver Prozess erfolgen. Das resultierende Differentialgleichungssystem ist beschrieben worden im Anhang zu der noch nicht veröffentlichten deutschen Patentanmeldung mit dem Aktenzeichen 10256315.2.

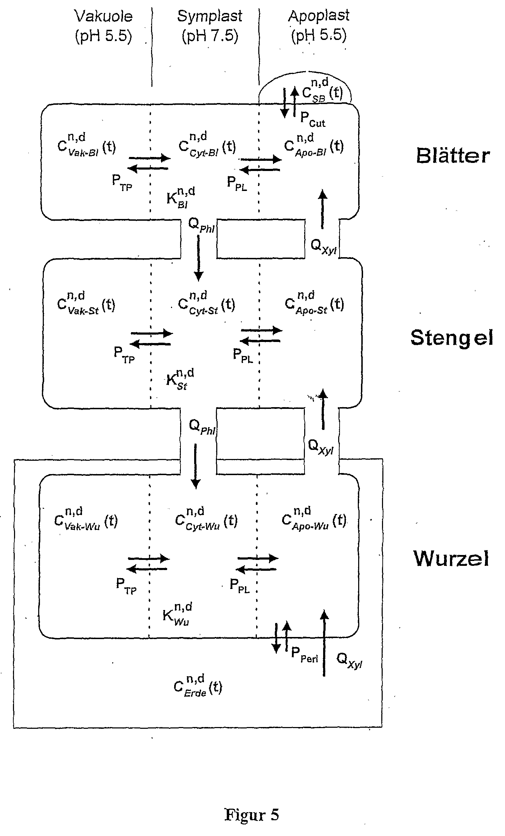

Das Pflanzenmodell aus Figur 5 stellt eine weitere bevorzugte Ausführungsform dar. Gemäß der typischen Pflanzenphysiologie beschreibt das Modell die Wurzeln, Stängel, und Blätter einer Pflanze. Jedes dieser Kompartimente besteht seinerseits aus drei Subkompartimenten, welche die Vakuole, den Symplasten und den Apoplasten repräsentieren. Diese Subkompartimente sind untereinander durch Membranen voneinander separiert. Diese Membranen können - wie in den vorab beschriebenen Organismen - auch hier durch passive Diffusion oder mittels aktivem Transport permeiert werden. Weiterhin sind die Subkompartimente durch unterschiedliche pH-Werte gekennzeichnet, welche insbesondere auf das Verteilungsverhalten von Säuren und Basen einen starken Einfluss ausüben. Zwischen den Kompartimenten verfügt die Pflanze über zwei Transportpfade: Der Xylemstrom im Apoplasten fließt von den Wurzel in Richtung Blätter, während der Phloemstrom umgekehrt Stoffe im Symplasten von den Blättern zu den Wurzeln transportiert.

### *Beispiele für Modellstrukturen*

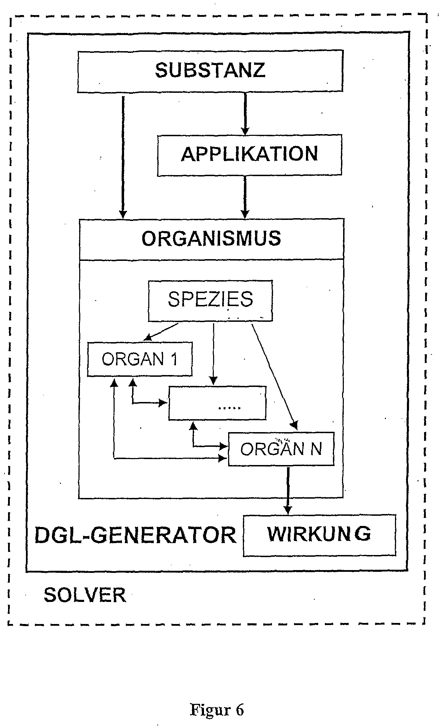

- Die einfachste Modellstruktur (Figur 6)

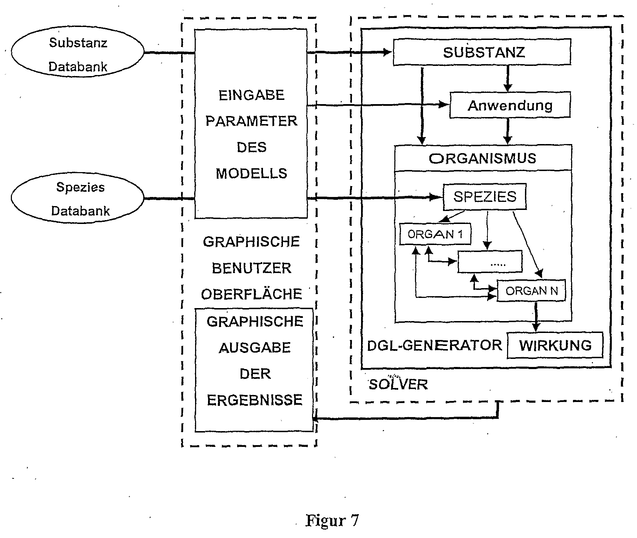

Figur 6 zeigt die einfachste Modellstruktur. Das komplette Modell besteht aus den Modulen "Substanz", "Verabreichung", "Organismus", "DGL-Generator", und "Integrator". Optional kann ein "Aktions-Modul" betrachtet werden. Figur 7 zeigt schematisch die Anbindung an eine oder mehrere Datenbanken, die z.B. die Substanzparameter oder die physiologischen und anatomischen Informationen des betrachteten Organismus beinhalten können.

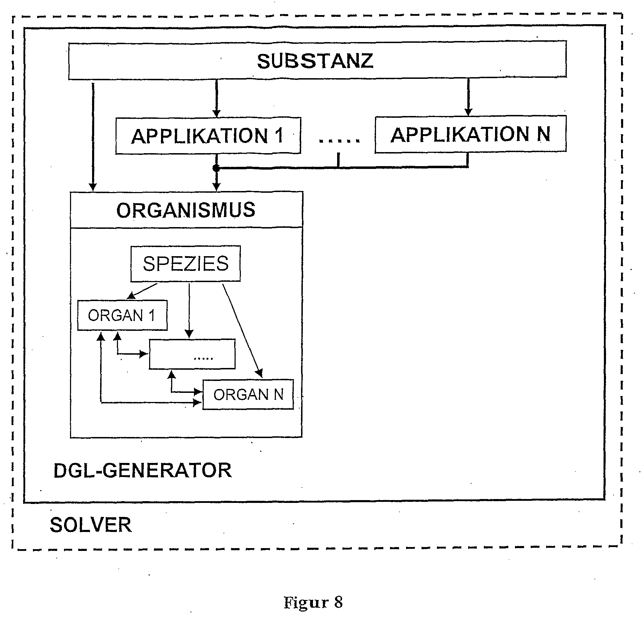

• Multiple Administration (Figur 8)

Die wiederholte Verabreichung eines Wirkstoffs an ein und denselben Organismus ist ein realistisches Szenario für eine Vielzahl von pharmazeutischen Substanzen, die in regelmäßigen Abständen über einen längeren Zeitraum genommen werden müssen wie z.B. antiinfektive Wirkstoffe. Mit diesem Beispielmodell können Langzeiteffekte wie z.B. die Akkumulation des Wirkstoffs in einzelnen Organen simuliert werden.

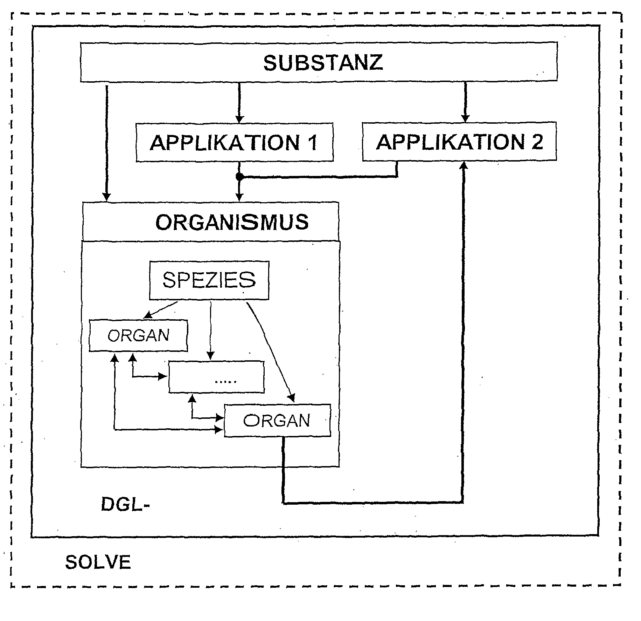

• Enterohepatischer Kreislauf (Figur 9)

Eine Reihe von chemischen Stoffen, die an Säugetiere verabreicht wurden, unterliegen einem enterohepatischen Kreislauf. In diesem Fall wird ein Teil der verabreichten Substanz unverändert in der Leber in die Gallenflüssigkeit abgesondert und darin in der Gallenblase (repräsentiert durch ORGAN N in Figur 9) akkumuliert. Ausgelöst durch einen chemischen Reiz, der z.B. durch die Einnahme einer Mahlzeit getriggert werden kann, zieht sich die Gallenblase zusammen und gibt ihren Inhalt - Gallenflüssigkeit, die unter anderem auch den Wirkstoff enthält - in den Zwölffingerdarm ab. Über den Darm kann die Substanz denn absorbiert werden und somit erneut Teil der systemischen Zirkulation werden. Im in Figur 8 skizzierten Modell kann dieser Prozess einfach als eine erneute intestinale Verabreichung (APPLICATION 2, charakterisiert durch eine Lag-Zeit) eines Teils der ursprünglichen Substanz beschrieben werden.

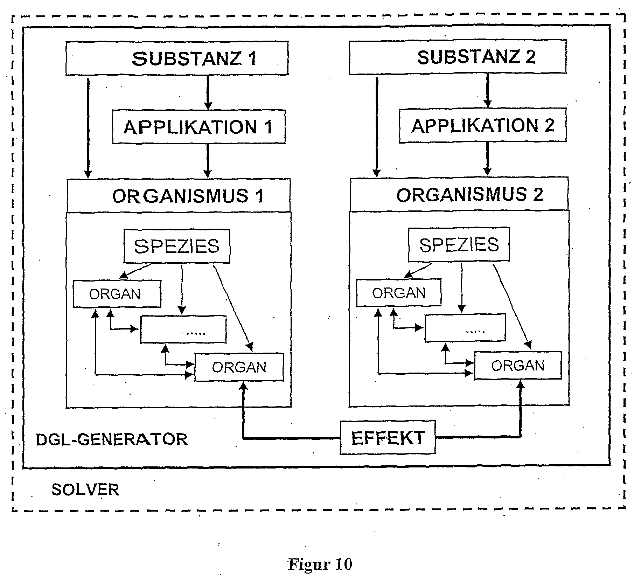

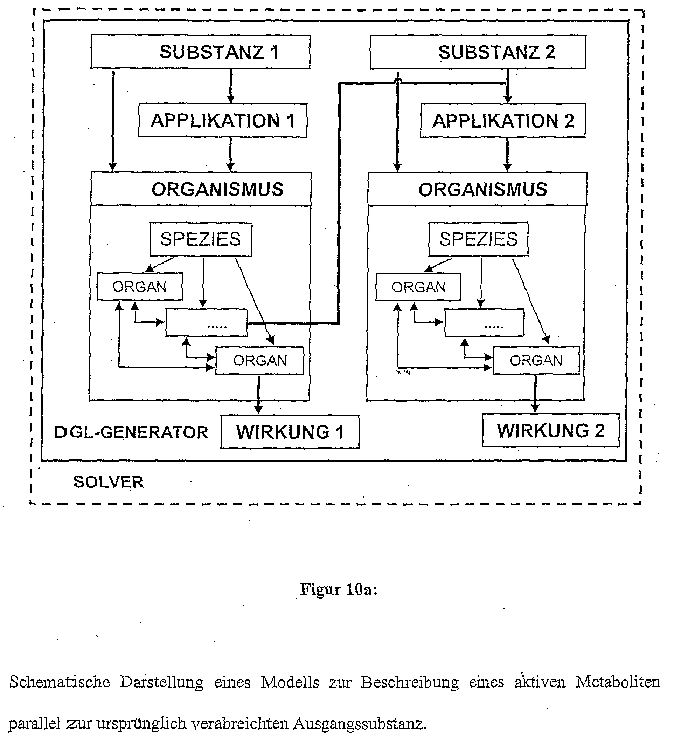

• Drug-Drug Wechselwirkung (Figur 10)

Wechselwirkungen von mehreren Substanzen (in diesem Beispiel: zwei Substanzen) untereinander sind von großer Bedeutung. Modellhaft kann dieser Fall wie folgt abgebildet werden: ORGANSIM 1 und 2 sind über identische physiologische Parameter definiert, denn sie repräsentieren ein und denselben Organismus. Zwei unterschiedliche Substanzen werden separat verabreicht, die Art, Zeitpunkt, und Dauer der Verabreichung kann für beide Substanzen unterschiedlich sein. Die eigentliche Wechselwirkung der beiden Substanzen wird in einem Aktionsmodul definiert, welches die Organe ORGAN N in den Organismen 1 und 2 verknüpft. Die Wechselwirkung kann beispielsweise eine kompetitive Inhibition oder jede andere denkbare biochemische Interaktion sein.

• Aktiver Metabolit / Prodrug (Abbildung 10a)

In vielen Fällen entsteht durch Metabolisierung der ursprünglich verabreichten Muttersubstanz z. B. im Darm oder in der Leber ein Stoffwechselprodukt, welches ebenfalls pharmakodynamisch wirksam ist ("aktiver Metabolit"). Somit zirkulieren zwei Substanzen zeitlich parallel in einem Organismus. Dieser Fall ist in Abbildung 10a skizziert. Die ursprünglich in der Form APPLIKATION 1 verabreichte Ausgangssubstanz SUBSTANZ 1 wird in einem Organ (z. B. in der Darmwand oder in der Leber) mit einer bestimmten Rate zu SUBSTANZ 1 umgewandelt. Mit der gleichen Rate entsteht also der Metabolit SUBSTANZ 2 in dem entsprechenden Organ. Dieser Prozess wird durch das Modul APPLIKATION 2 beschrieben. Beide Substanzen können an demselben oder auch an unterschiedlichen Targets wirken (WIRKUNG 1 und 2). Ein in diesem Beispiel enthaltener Sonderfall ist das sog. Prodrug-Konzept: Bei diesem Konzept wird eine zunächst unwirksame Ausgangssubstanz (Prodrug) verabreicht, die erst im Körper durch Metabolisierung in die aktive Substanz umgewandelt wird (d. h. WIRKUNG 1 = 0).

• Mutter-Foetus Model (Figur 11)

Das Mutter-Foetus Modell in Figur 11 ist ein Beispiel, bei dem zwei unterschiedliche aber gekoppelte Organismen simultan modelliert werden. ORGANISM 1 repräsentiert die Mutter und ORGANISM 2 den Foetus. Die Blutzirkulation des Feten ist an den Blutkreislauf der Mutter über die Plazenta (hier repräsentiert durch ORGAN N in ORGANISM 1) angeschlossen. In diesem Beispiel ist von großer Bedeutung, dass die physiologischen Parameter, insbesondere die Organvolumina und Blutflussraten des Feten, Funktionen der Zeit sein können, um das genaue Gestationsalter sowie das Wachstum des Feten im Mutterleib berücksichtigen zu können.

• Kombinierte Pflanzen/Insekt Modelle (Figur 11)

Ähnlich wie das Mutter-Foetus-Modell stellt auch das Pflanze/Insekt Modell eine Kombination zweier gekoppelter Organismen dar, allerdings sind die Organismen hier physiologisch unterschiedlich. ORGANISM 1 beschreibt eine Pflanze, die eine applizierte Substanz, z.B. ein Insektizid, über die Blattkutikeln oder die Wurzeln aufnimmt. Nach Verteilung im gesamten Organismus steht diese Substanz dann auch in den übrigen Kompartimenten zur Verfügung. Durch Fraß an einem Blatt, welches ein spezielles Kompartiment in dem Pflanzen-Organismus darstellt, kann die Substanz von einem Insekt (ORGANISM 2) aufgenommen werden, verteilt sich dort und erzielt schließlich im Zielorgan, beispielsweise dem Nervensystem der Raupe, seine insektizide Wirkung.

• Weitere Kombinationen der vorab beschriebenen Beispiele

Kombinationen der vorab beschriebenen Beispiele sind ebenfalls von großer Bedeutung. Die multiple Administration von zwei oder mehreren miteinander wechselwirkenden Substanzen in einem Mutter-Foetus-Modell z.B. kann zur frühzeitigen Abschätzung des toxikologischen Risikos des Feten herangezogen werden.

• Simulationsergebnisse für das Ganzkörper-Säugetiermodell

Im folgenden wird beispielhaft gezeigt, welche Schritte zur Durchführung einer Simulation erforderlich sind. Zunächst müssen die substanzabhängigen Eigenschaften bestimmt werden. Als Beispiel dient hier eine Substanz X mit den folgenden Eigenschaften:

Tabelle 1: Substanzabhängige Parameter der Substanz X

| Parameter | Wert | Einheit |
|---|---|---|
| Lipophilie (LogMA) | 2.7 | -/- |
| ungebundene Plasmafraktion | 0.25 | -/- |
| Leberclearance | 1 | ml/min/kg |
| Dosis | 1 | mg/kg |

Aus der Lipophilie (MA = 10^LogMA) und der ungebundenen Plasmafraktion ($f_u$) können dann mit Hilfe von veröffentlichten Gleichungen die Organ-Verteilungskoeffizienten berechnet werden [M. Härter, J. Keldenich, W. Schmitt: Estimation of physicochemical and ADME parameters, Ch. 26 in: Combinatorial Chemisrty - A Practical Handbook, Part IV. Eds. K. C. Nicolau et al., Wiley VCH, Weinheim, 2002]. Für die Organ-Verteilungskoeffizienten ergeben sich in diesem Beispiel die folgenden Werte:

Tabelle 2: Aus den Werten von Tabelle 1 resultierende Verteilungskoeffizienten [8].

| Organ/Plasma | Verteilungskoeffizient |
|---|---|
| Magen | 8.34 |
| Dünndarm | 8.34 |
| Dickdarm | 8.34 |
| Pankreas | 10.57 |
| Milz | 2.74 |
| Leber | 9.35 |
| Niere | 7.19 |
| Lunge | 1.97 |
| Gehirn | 14.21 |
| Herz | 13.28 |
| Muskel | 2.33 |
| Knochen | 34.45 |
| Haut | 13.49 |
| Fett | 100.42 |
| Hoden | 4.42 |

Weiterhin müssen die physiologischen Parameter wie Organvolumina, -blutflussraten und Zusammensetzung hinsichtlich vaskulärem, interstitiellen und zellulärem Raum bekannt sein. Diese sind ebenfalls in der Literatur beschrieben. Für die Spezies Maus, Ratte, Hund und Mensch finden sich beispielsweise die in Tabellen 3 bis 5 aufgelisteten Werte:

Tabelle 3: Organvolumina für Maus, Ratte, Hund und Mensch (Literaturdaten)

| Organvolumina [ml] | MAUS | RATTE | HUND | MENSCH |
|---|---|---|---|---|
| venöser Blutpool | 0.128 | 6.8 | 0.8 | 250 |

(fortgesetzt)

| Organvolumina [ml] | MAUS | RATTE | HUND | MENSCH |
|---|---|---|---|---|
| arterieller Blutpool | 0.073 | 2.9 | 0.8 | 140 |
| Lunge | 0.201 | 2.2 | 145 | 670 |
| Magen | 0.1 | 1.1 | 40 | 150 |
| Dünndarm | 0.2 | 11.1 | 140 | 640 |
| Dickdarm | 0.2 | 11.1 | 140 | 370 |
| Pankreas | 0.13 | 1.3 | 10 | 100 |
| Milz | 0.13 | 1.3 | 10 | 180 |
| Leber | 0.941 | 10 | 366 | 1710 |
| Gallenblase | 0.12 | 1.2 | 10 | 20 |
| Niere | 0.449 | 7 | 154 | 720 |
| Gehirn | 0.336 | 1.671 | 90.4 | 1486 |
| Herz | 0.5 | 1.2 | 90 | 330 |
| Muskel | 11.74 | 110.1 | 6502 | 30200 |
| Knochen | 2.775 | 28.2 | 2584 | 12060 |
| Haut | 5.16 | 43.4 | 647 | 3020 |
| Fett | 1.51 | 14.2 | 2670 | 10060 |
| Hoden | 0.001 | 2.5 | 5 | 35 |
| Summe der Organe | 24.694 | 257.271 | 13605 | 62141 |

Tabelle 4: Organblutflussraten für Maus, Ratte, Hund und Mensch (Literaturdaten)

| Blutfluss [ml/min] | MAUS | RATTE | HUND | MENSCH |
|---|---|---|---|---|
| Magen | 0.5 | 1.22 | 40 | 60 |
| Dünndarm | 0.5 | 7.02 | 400 | 600 |
| Dickdarm | 0.5 | 3.82 | 160 | 240 |
| Pankreas | 0.005 | 0.51 | 40 | 60 |
| Milz | 0.063 | 0.63 | 120 | 180 |
| Leber | 2.25 | 5.5 | 310 | 390 |
| Niere | 0.67 | 14.6 | 243 | 1133 |
| Gehirn | 0.11 | 1.1 | 145 | 700 |
| Herz | 0.039 | 3.92 | 180 | 240 |
| Muskel | 0.33 | 7.2 | 118 | 550 |
| Knochen | 0.007 | 1.6 | 35 | 167 |
| Haut | 0.02 | 4.8 | 10 | 50 |
| Fett | 0.002 | 1.8 | 3 | 300 |
| Hoden | 0.0002 | 0.48 | 0.66 | 2.6 |
| Lunge (=Summe) | 4.996 | 53.72 | 1805 | 4670 |

Tabelle 5: Zusammensetzung der Organe von Säugetieren (Literaturdaten)

| Volumenanteil | f_vas | f_int | f_cell |
|---|---|---|---|
| Fettgewebe | 0.010 | 0.135 | 0.855 |
| Gehirn | 0.037 | 0.004 | 0.959 |
| Magen-Darm-Trakt | 0.032 | 0.100 | 0.868 |
| Herz | 0.262 | 0.100 | 0.638 |
| Niere | 0.105 | 0.200 | 0.695 |
| Leber | 0.115 | 0.163 | 0.722 |

(fortgesetzt)

| Volumenanteil | f_vas | f_int | f_cell |
|---|---|---|---|
| Lunge | 0.626 | 0.188 | 0.186 |
| Muskel | 0.026 | 0.120 | 0.854 |
| Knochen | 0.041 | 0.100 | 0.859 |
| Haut | 0.019 | 0.302 | 0.679 |
| Bauchspeicheldrüse | 0.180 | 0.120 | 0.700 |
| Milz | 0.282 | 0.150 | 0.568 |
| Hoden | 0.140 | 0.069 | 0.791 |

Im Folgenden sind Simulationsergebnisse gezeigt, die mit dem Ganzkörpermodell nach Figur 3 erzielt wurden. Hierbei wurde als einfachster Grenzfall angenommen, dass der Transport in die Organe blutflusslimitiert ist (d. h. $PA^{org} \rightarrow \infty$).

Figur 12 zeigt die resultierenden Organkonzentrationen in der Ratte nach einer intravenösen Bolus Administration von 1 mg/kg der Substanz X.

Figur 13 zeigt die Konzentration-Zeit-Kurve im Plasma simuliert in den vier verschiedenen Spezies nach einer intravenösen Bolus Administration von 1 mg/kg der Substanz X.

Figur 14 zeigt die Konzentrations-Zeit Kurven in den diversen Organen eines Menschen nach mulitpler intravenöser Bolus Administration von 1 mg/kg der Substanz X über drei Tage (Dosierungsschema: 6 h - 6 h - 12 h, berechnet nach dem Modell in Figur 8)

Figur 15 zeigt Konzentration-Zeit-Kurve im Plasma nach peroraler Administration von 1 mg/kg der Substanz X im Menschen ohne enterohepatischem Kreislauf und mit enterohepatischem Kreislauf berechnet nach dem Modell in Figur 9 (der Anteil der biliären Clearance an der totalen ist mit 25 % angenommen).

**Patentansprüche**

1. Computer implementiertes Verfahren zur Ermittlung der Wechselwirkung einer oder mehrerer chemisch wirksamer Substanzen mit einem oder mehreren Organismen mittels eines Computersystems umfassend mindestens ein Substanz-Modul, ein Applikations-Modul und ein Organismus-Modul, wobei das Substanzmodul physiko-chemische und/oder biochemische Informationen über die Substanz(en), dessen/deren Verhalten simuliert werden soll, enthält, das Organismus-Modul physiologische und anatomische Informationen, die den Organismus charakterisieren, mit dem die Substanz wechselwirken soll, beinhaltet,
das Applikations-Modul Informationen über den Ort und das zeitliche Profil der Verabreichung der Substanz(en) beinhaltet,
und das Verfahren folgende Schritte umfasst:

A) Auswahl mindestens einer Substanz aus dem Substanzmodul und Analyse ihrer physiko-chemischen und/oder biochemischen Eigenschaften,
B) Aus dem Organismus-Modul Auswahl anatomischer und physiologischer Kompartimente des jeweiligen Organismus ausgewählt wenigstens aus: a) im Falle von Säugetieren oder Insekten: Lunge periphere Organe, Blutwege, bevorzugt arterielle und/oder venöse Blutwege, oder Blutflüssigkeit; b) im Falle von Pflanzen Xylem- und Phloemstrom und Wurzel und /oder Blatt und/oder Stängel,
C) Beschreibung der Kompartimente in einem Rechenprogramm im Hinblick auf die Transportprozesse der Substanz von und zu den Kompartimenten, Abbauprozesse und Wirkprozesse durch Massentransportgleichungen und kinetische Reaktionsgleichungen zur Beschreibung des jeweiligen Prozesses,
D) Beschreibung der Transportprozesse und gegebenenfalls der Abbauprozesse der wirksamen Substanz im Organismus im Rechenprogramm durch Massentransportgleichungen und kinetische Reaktionsgleichungen,
E) Verknüpfung der nach Schritt C) und D) ausgewählten Gleichungssystemen und numerische Berechnung des entstehenden Systems gekoppelter Differentialgleichungen,
F) Bestimmung des Konzentrations-/Zeitverlaufs der Wirksubstanz in ausgewählten Kompartimenten,

**dadurch gekennzeichnet, dass**:

- für das Rechenprogramm eine hierarchische Modell-Architektur bestehend aus Modulen, die unterschiedliche Funktionalitäten aufweisen, verwendet wird und mindestens ein das Substanz-Modul, das Applikations-Modul

**14**

und das Organismus-Modul umfassen,

- dass die Module dynamisch während der Laufzeit des Verfahrens und hierarchisch durch das Computersystem miteinander verbunden werden,

- dass anschließend im Schritt E) ein System von gekoppelten Differentialgleichungen durch einen DGL-Erzeuger automatisch erzeugt wird, welches durch numerische Integration durch einen numerischen Solver gelöst wird und

- dass als Ergebnis Konzentrations/Zeit-Kurven der betrachteten Substanz(en) in Kompartimenten des betrachteten Organismus geliefert werden

2. Verfahren nach Anspruch 1, **dadurch gekennzeichnet, dass** der Organismus bzw. die Organismen Säugetiere, aus der Reihe Mensch, Affe, Hund, Schwein, Ratte oder Maus, oder Insekten, insbesondere Raupen, oder Pflanzen darstellen.

3. Verfahren nach Anspruch 1 oder 2, **dadurch gekennzeichnet, dass** der Wirkstoff intravenös, oral, topikal, subkutan, intraperitoneal, inhalativ über die Nase oder Lunge, oder intracerebrovaskulär (im Fall von Säugetieren), bzw. intra-haemolymphatisch, topikal, oder oral durch Fraß oder Schlundsonde (im Fall von Insekten) appliziert wird, oder die Applikation als Spritzbrühe mit Aufnahme über Blätter oder Wurzeln (im Fall von Pflanzen) erfolgt.

4. Ein Verfahren nach den Ansprüchen 1 bis 3, **dadurch gekennzeichnet, dass** die physiologischen Parameter, die die Organismen beschreiben, zeitabhängige Parameter sind.

5. Verfahren nach den Ansprüchen 1 bis 4, **dadurch gekennzeichnet, dass** die Parameter teilweise einer Datenbank entnommen werden, die an das Rechner-System angebunden ist.

6. Ein Verfahren nach den Ansprüchen 1 bis 4, **dadurch gekennzeichnet, dass** die physiologischen und anatomischen Parameter mittels Zufallszahlen im Rahmen einer vorgegebenen statistischen Streuung variiert werden.

7. Verfahren nach einem der Ansprüche 1 bis 6, **dadurch gekennzeichnet, dass** mehrere Modelle von Organismen zu einem Gesamtmodell, insbesondere zur Simulation von Drug-Drug-Wechselwirkungen, Mutter-Foetus-Modell, kombiniertes Insekt-Pflanzen-Modell, zusammengefasst werden.

## Claims

1. Computer-implemented method for determining the interaction of one or more chemical active compounds with one or more organisms by means of a computer system comprising at least one substance module, an application module and an organism module, wherein the substance module contains physico-chemical and/or biochemical information on the substance(s) whose behaviour is to be simulated,

the organism module comprises physiological and anatomical information which characterizes the organism with which the substance is to interact,

the application module comprises information on the location and the time profile of the administration of the substance(s), and the method comprises the following steps:

A) selection of at least one substance from the substance module and analysis of its physico-chemical and/or biochemical characteristics,

B) selection from the organism module of anatomical and physiological compartments of the organism in question, selected from at least amongst: a) in the case of mammals or insects: the lungs, peripheral organs, blood vessels, preferably arteries and/or veins, or blood fluid; b) in the case of plants: xylem and phloem stream, and root and/or leaf and/or stem,

C) description of the compartments in a computing program with regard to the transport processes of the substance from and to the compartments, degradation processes and active processes by mass transportation equations and kinetic reaction equations for describing the process in question,

D) description of the transport processes and, if appropriate, the degradation processes of the active compound of the organism, in the computing program by mass transportation equations and kinetic reaction equations,

E) combination of the equation systems selected in steps C) and D) and numerical calculation of the resulting system of coupled differential equations,

F) determination of the concentration-time curve of the active compound in selected compartments,

**characterized in that**:

- a hierarchical model architecture consisting of modules with different functionalities is used for the computing program and comprise at least the substance module, the application module and the organism module,
- the modules are interconnected dynamically during the execution time of the program and hierarchically by the computer system,
- subsequently, in step E), a system of coupled differential equations is generated automatically by a DEQ generator, which is solved by numeric integration by a numeric solver and
- as a result, concentration/time curves of the respective substance(s) in the diverse compartments of the organism in question are provided.

2. Method according to Claim 1, **characterized in that** the organism(s) represent(s) mammals from the group consisting of man, monkey, dog, pig, rat or mouse, or insects, in particular caterpillars, or plants.

3. Method according to Claim 1 or 2, **characterized in that** the active compound is applied via the intravenous, oral, topical, subcutaneous, intraperitoneal route, the inhalative route via the nose or the lungs, or the intracerebrovascular route (in the case of mammals), or via the intrahaemolymphatic, topical or oral route via feeding or gavage (in the case of insects), or else in the form of a spray mixture with foliar uptake or uptake via the roots (in the case of plants).

4. Method according to Claims 1 to 3, **characterized in that** the physiological parameters which describe the organisms are time-dependent parameters.

5. Method according to Claims 1 to 4, **characterized in that** some of the parameters are taken from a database which is linked to the computer system.

6. Method according to Claims 1 to 4, **characterized in that** the physiological and anatomical parameters are varied within a predetermined statistical variation by means of random numbers.

7. Method according to one of Claims 1 to 6, **characterized in that** a multiplicity of models of organisms are combined in an integrated model, in particular for the simulation of drug-drug interactions, mother-foetus model, combined insect-plant model.

**Revendications**

1. Procédé informatisé pour la détermination de l'interaction d'une ou plusieurs substances chimiquement efficaces avec un ou plusieurs organismes, à l'aide d'un système informatique comprenant au moins un module substance, un module application et un module organisme, dans lequel
le module substance contient des informations physico-chimiques et/ou biochimiques concernant la ou les substances dont il s'agit de simuler le comportement, le module organisme comprend des informations physiologiques et anatomiques qui caractérisent l'organisme avec lequel la substance doit interagir,
le module application comprend des informations sur le lieu et le profil temporel de l'administration de la ou des substances,
et le procédé comprend les étapes suivantes :

A) choix d'au moins une substance à partir du module substance, et analyse de ses propriétés physico-chimiques et/ou biochimiques,
B) à partir du module organisme, choix de compartiments anatomiques et physiologiques de l'organisme considéré, choisis au moins parmi : a) dans le cas des mammifères ou des insectes : le poumon, les organes périphériques, les voies sanguines, de préférence les voies sanguines artérielles et/ou veineuses, ou le plasma sanguin ; b) dans le cas des plantes, le flux xylémique et phloémique, et les racines et/ou la feuille et/ou la tige,
C) description des compartiments dans un programme de calcul, pour ce qui concerne les processus de transport de la substance à partir de compartiments et allant vers eux, les processus de dégradation et les processus d'action, par des équations du transport de masse et des équations de réaction cinétique pour la description du processus considéré,
D) description des processus de transport et éventuellement des processus de dégradation de la substance active dans l'organisme dans le programme de calcul par les équations de transport de masse et les équations de réaction cinétique,

E) liaison des systèmes d'équations choisis dans les étapes C) et D) et du calcul numérique du système obtenu des équations différentielles couplées,

F) détermination de la courbe concentration/temps du principe actif dans les compartiments choisis,

**caractérisé en ce que**

- pour le programme de calcul, on utilise une architecture à modèle hiérarchique, constitué de modules qui présentent des fonctionnalités différentes, et comprennent au moins le module substance, le module application et le module organisme,

- les modules sont dynamiquement reliés les uns aux autres pendant la durée d'exploitation du procédé, et d'une manière hiérarchique grâce au système informatique,

- après l'étape E), il y a production automatique d'un système d'équations différentielles couplées, grâce à un générateur d'équations différentielles, qui est résolu grâce à une intégration numérique faisant appel à un solveur numérique, et

- on obtient en résultat les courbes concentration/temps de la ou des substances considérées dans les compartiments de l'organisme considéré.

2. Procédé selon la revendication 1, **caractérisé en ce que** l'organisme ou les organismes représentent des mammifères de la série humain, singe, chien, porc, rat ou souris, ou des insectes, en particulier des chenilles, ou des plantes.

3. Procédé selon la revendication 1 ou 2, **caractérisé en ce que** le principe actif est administré par voie intraveineuse, orale, topique, sous-cutanée, intrapéritonéale, par inhalation par le nez ou les poumons, ou par voie intra-cérébrovasculaire (dans le cas des mammifères), et par voie intra-hémolymphatique, topique, ou orale par l'intermédiaire de l'alimentation ou d'une sonde pharyngienne (dans le cas d'insectes), ou encore l'application se fait sous forme d'une bouillie de pulvérisation, avec réception sur les feuilles ou les racines (dans le cas des plantes).

4. Procédé selon les revendications 1 à 3, **caractérisé en ce que** les paramètres physiologiques qui décrivent les organismes sont des paramètres dépendant du temps.

5. Procédé selon les revendications 1 à 4, **caractérisé en ce que** les paramètres sont en partie prélevés d'une banque de données qui est raccordée au système de calcul.

6. Procédé selon les revendications 1 à 4, **caractérisé en ce qu'**on fait varier les paramètres physiologiques et anatomiques à l'aide de nombres aléatoires dans le cadre d'une dispersion statistique prédéfinie.

7. Procédé selon l'une des revendications 1 à 6, **caractérisé en ce que** plusieurs modèles d'organismes sont regroupés en un modèle global, en particulier pour la simulation d'interactions médicament-médicament, d'un modèle mère-foetus, d'un modèle combiné insecte-plante.

VENÖSER BLUTPOOL

$Q^{lung}$,

LUNGE

$Q^{lung}$,

ARTERIELLER BLUTPOOL

$Q^{org}$,

PERIPHERES ORGAN

$CL^{org}$ (ggf.)

Figur 1

Figur 2

**Figur 3**

Figur 4

Vakuole (pH 5.5) | Symplast (pH 7.5) | Apoplast (pH 5.5)

$C_{SB}^{n,d}(t)$

$P_{Cut}$

$C_{Vak\text{-}Bl}^{n,d}(t)$  $C_{Cyt\text{-}Bl}^{n,d}(t)$  $C_{Apo\text{-}Bl}^{n,d}(t)$

$P_{TP}$  $P_{PL}$

$K_{Bl}^{n,d}$

**Blätter**

$Q_{Phl}$  $Q_{Xyl}$

$C_{Vak\text{-}St}^{n,d}(t)$  $C_{Cyt\text{-}St}^{n,d}(t)$  $C_{Apo\text{-}St}^{n,d}(t)$

$P_{TP}$  $P_{PL}$

$K_{St}^{n,d}$

**Stengel**

$Q_{Phl}$  $Q_{Xyl}$

$C_{Vak\text{-}Wu}^{n,d}(t)$  $C_{Cyt\text{-}Wu}^{n,d}(t)$  $C_{Apo\text{-}Wu}^{n,d}(t)$

$P_{TP}$  $P_{PL}$

$K_{Wu}^{n,d}$

**Wurzel**

$P_{Peri}$  $Q_{Xyl}$

$C_{Erde}^{n,d}(t)$

Figur 5

Figur 6

Figur 7

Figur 8

Figur 10

Figur 10a:

Schematische Darstellung eines Modells zur Beschreibung eines aktiven Metaboliten parallel zur ursprünglich verabreichten Ausgangssubstanz.

Figur 11

**Figur 12**

| VEN | Venöser Blutpool |
|-----|------------------|
| LNG | Lunge |
| MUS | Muskel |
| FAT | Fett |
| SKN | Haut |
| LIV | Leber |
| STO | Magen |
| SIN | Dünndarm |
| LIN | Dickdarm |
| PAN | Bauchspeicheldrüse |
| SPL | Milz |
| KID | Niere |
| BRN | Gehirn |
| HRT | Herz |
| BON | Knochen |
| TST | Hoden |

Figur 13

**Figur 14**

| VEN | Venöser Blutpool |
|-----|------------------|
| LNG | Lunge |
| MUS | Muskel |
| FAT | Fett |
| SKN | Haut |
| LIV | Leber |
| STO | Magen |
| SIN | Dünndarm |
| LIN | Dickdarm |
| PAN | Bauchspeicheldrüse |
| SPL | Milz |
| KID | Niere |
| BRN | Gehirn |
| HRT | Herz |
| BON | Knochen |
| TST | Hoden |

**Figur 15**

EHC   Enterohepatischer Kreislauf

**EP 1 671 251 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5930154 A
- WO 0065523 A
- DE 10256315

**In der Beschreibung aufgeführte Nicht-Patentliteratur**

- **R. KAWAI ; M. LEMAIRE ; J. L. STEIMER ; A. BRUELISAUER ; W. NIEDERBERGER ; M. ROWLAND.** Physiologically based pharmacokinetic study on a Cyclosporin derivate, SDZ IMM 125. *J. Pharmacokin. Biopharm.,* 1994, vol. 22, 327-365
- **P. POULIN ; F.P. THEIL.** Prediction of pharmacokinetics prior to in vivo studies. 1. Mechanism-based prediction of volume of distribution. *J. Pharm. Sci.,* 2002, vol. 291, 129-156
- **P. POULIN ; F.P. THEIL.** Prediction of Pharmacokinetics prior to in vivo studies. II. Generic physiologically based pharmacokinetic models of drug disposition. *J. Pharm. Sci.,* 2002, vol. 91, 1358-1370
- **R. GREENWOOD ; M. G. FORD ; E. A. PEACE ; D. W. SALT.** The Kinetics of Insecticide Action. Part IV: The in vivo Distribution of Pyrethroid Insecticides during Insect Poisoning. *Pestic. Sci.,* 1990, vol. 30, 97-121
- **N. M. SATCHIVI ; E. W. STOLLER ; L. M. WAX ; D. P. BRISKIN.** A nonlinear dynamic simulation model for xenobiotik transport and whole plant allocation following foliar application. *Pestic. Biochem. Physiol.,* 2000, vol. 68, 67-84

- **NESTOROV IVAN.** Whole body pharmacokinetic models. *CLINICAL PHARMACOKINETICS,* 18. August 2003, vol. 42 (10), 883-908
- **KLEIN MICHAEL T et al.** BioMOL: a computer-assisted biological modeling tool for complex chemical mixtures and biological processes at the molecular level. *ENVIRONMENTAL HEALTH PERSPECTIVES,* Dezember 2002, vol. 110 (6), 1025-1029
- THE KINETICS OF INSECTICIDE ACTION. PART IV: THE IN-VIVO DISTRIBUTION OF PYRETHROID INSECTICIDES DURING INSECT POISONING. **GREENWOOD R et al.** PESTICIDE SCIENCE. ELSEVIER APPLIED SCIENCE PUBLISHER, 1990, vol. 30, 97-121
- **BIRTA L G et al.** SIMPAK-an interactive/graphics program for continuous-simulation system. *SIMULATION USA,* Oktober 1976, vol. 37 (4), ISSN 0037-5497, 115-122
- Estimation of physicochemical and ADME parameters. **M. HÄRTER ; J. KELDENICH ; W. SCHMITT et al.** Combinatorial Chemisrty - A Practical Handbook. Wiley VCH, 2002